# EUROPÄISCHE PATENTANMELDUNG

(11) **EP 1 911 410 A1**
(43) Veröffentlichungstag der Anmeldung: **16.04.2008**
(21) Anmeldenummer: 06021252.9
(22) Anmeldetag: 10.10.2006
(51) Int. Cl.: A61B 18/20

(54) **Vorrichtung für dermatologische Behandlungen**

(71) Anmelder: Wavelight Aesthetic GmbH, 91058 Erlangen (DE)
(72) Erfinder: Fischer, Dietmar, 91056 Erlangen (DE); Haberer, Daniel, 91301 Forchheim (DE); Kaukel, Sebastian, 91334 Hemhofen (DE); Parusel, Henry, 06386 Quellendorf (DE); Pribbernow, Arnold, 91245 Simmelsdorf (DE); Schmidt, Udo, 90562 Heroldsberg (DE); Schulze, Stefan, 90491 Nürnberg (DE); Winkler, Martin, 09337 Hohenstein-Ernsttal (DE)
(74) Vertreter: von Hellfeld, Axel

(57) **Zusammenfassung**

Eine Vorrichtung für dermatologische Behandlungen hat ein Basisgerät 10, an das wahlweise über flexible Leitungen 12 unterschiedliche Applikationsköpfe 14 anschließbar sind zum Aufbringen von Strahlung insbesondere einer LED und eines Lasers oder einer hochenergetischen Blitzlampe.

## Beschreibung

Die Erfindung betrifft eine Vorrichtung für dermatologische Behandlungen, mit der Haut mit elektromagnetischer Strahlung bestrahlt werden kann.

Eine solche Vorrichtung ist z.B. aus der US 6,273,884 B1 bekannt. Dort wird ein Applikationskopf vom Operateur über die zu behandelnde Haut geführt. Auch die WO 2004/004831 A1 beschreibt ein ähnliches System.

Bei den bekannten Systemen für dermatologische Hautbehandlungen mit elektromagnetischer Strahlung ist ein sogenanntes Basisgerät (Basisstation) vorgesehen und der Arzt kann über eine flexible (biegsame) Leitung einen sogenannten Applikationskopf mit dem Basisgerät verbinden. Eine Strahlungsquelle kann entweder direkt im Applikationskopf angeordnet sein oder auch im Basisgerät. Im letztgenannten Fall enthält dann die flexible Leitung einen Lichtleiter, um die im Basisgerät erzeugte Strahlung zum Applikationskopf zu übertragen.

Die Erfindung setzt sich das Ziel, eine Vorrichtung für dermatologische Behandlungen bereitzustellen, die mit relativ geringem apparativen Aufwand eine Hautbehandlung mit unterschiedlicher Strahlung ermöglicht.

Hierzu stellt die Erfindung eine Vorrichtung für dermatologische Behandlungen bereit mit einem Basisgerät, an das wahlweise über flexible Leitungen zumindest ein erster Applikationskopf anschließbar ist zum Aufbringen von Strahlung einer LED auf Haut, und/oder zumindest ein zweiter Applikationskopf zum Aufbringen von Strahlung eines Lasers oder einer hochenergetischen Blitzlampe auf Haut.

Die Erfindung ermöglicht also mit einem einzigen Basisgerät den wahlweisen Anschluss eines ersten Applikationskopfes zum Bestrahlen von Haut mit von einer oder mehreren LEDs emittierter Strahlung und/oder das Anschließen eines zweiten Applikationskopfes über eine andere flexible Leitung, sodass damit Strahlung entweder eines Lasers oder einer hochenergetischen Blitzlampe auf Haut aufbringbar ist.

Ein Arzt oder Hospital braucht damit für die Behandlung der Haut mit unterschiedlicher elektromagnetischer Strahlung nicht für jede Strahlungsart unbedingt ein eigenes Basisgerät zu erwerben und vorzuhalten, vielmehr dient ein einziges Basisgerät zur Versorgung und Steuerung unterschiedlicher Applikationsköpfe mit jeweils unterschiedlichen Arten elektromagnetischer Strahlung.

Damit ist es dem Arzt ermöglicht, mit einem einzigen, apparativ relativ wenig aufwändigen Gerätesystem unterschiedliche medizinische Behandlungen durchzuführen. Entsprechendes gilt für einen Kosmetiker.

Zu dem damit ermöglichten großen Anwendungsspektrum dermatologischer Behandlungen gehören zum Beispiel die Epilation (Haarentfernung) oder eine PDT (fotodynamische Therapie). Diese Behandlungen erfordern unterschiedliche Strahlungsarten (Wellenlänge, Energie, Zeit, Zusatzeinrichtungen etc.). Auch wird bei dem erfindungsgemäßen System die Bedienung vereinfacht, da für alle Applikationen dasselbe Basisgerät mit denselben Bedienungselementen eingesetzt wird. Ein weiterer Vorteil des erfindungsgemäßen System liegt darin, dass ein Benutzer zu einem gegebenen Basisgerät wahlweise Applikationsköpfe nachrüsten kann.

Das erfindungsgemäße Basisgerät ermöglicht es auch, über eine Faserkopplung Licht- oder Laserquellen anzuschließen, die dann für andere Behandlungen im Körper (z.b. HNO, Chirurgie, Urologie, Kardiologie) eingesetzt werden können.

Gemäß einer Ausgestaltung der Erfindung sind die LED und/oder der Laser jeweils in einem zugeordneten Applikationskopf angeordnet.

Eine andere Ausgestaltung der Erfindung sieht vor, dass die LED und/oder der Laser und/oder die Blitzlampe in dem genannten Basisgerät angeordnet sind, sodass die Strahlung mittels Lichtleiter zu den jeweils vorgesehenen Applikationsköpfen übertragbar ist.

Es können auch mehrere der genannten Strahlungsquellen gleicher Art jeweils in dem Basisgerät oder dem Applikationskopf angeordnet sein.

Bevorzugt wird eine schmalbandig emittierende LED eingesetzt.

Eine andere bevorzugte Ausgestaltung sieht vor, dass die LED mit Intensitäten emittiert, die ausgelegt sind, fotobiologische Effekte in der Haut zu erzielen.

Wenn hier von LED die Rede ist, erfasst dies auch OLEDs (organische lichtemittierende Dioden).

Eine weitere bevorzugte Ausgestaltung der Erfindung sieht vor, dass die LED oder mehrere LEDs im UVA-Bereich emittieren. Es können auch LEDs vorgesehen sein, die im UVB-Bereich emittieren.

Eine andere Variante der Erfindung sieht vor, dass in einem Applikationskopf zwei oder mehr LEDs angeordnet sind, die mit unterschiedlichen Wellenlängen emittieren, insbesondere zur homogenen Beleuchtung einer zu behandelnden Hautfläche.

Es können auch Applikationsköpfe vorgesehen werden, die es ermöglichen, unterschiedliche LEDs wahlweise einzusetzen.

Der erfindungsgemäß neben den LEDs eingesetzte Laser kann insbesondere ein Festkörperlaser, ein Diodenlaser, und weiter insbesondere eine gepulster IR-Laser sein mit Intensitäten jeweils zur Erzielung von fotothermischen Effekten in der Haut. Dabei kann der Festkörperlaser bevorzugt ein CW-Laser sein.

Eine andere bevorzugte Ausgestaltung der Erfindung sieht einen Applikationskopf vor mit Einrichtungen zum Aufbringen einer Flüssigkeit oder Creme auf die Haut. Ein solcher Applikationskopf ist auch eigenständig Gegenstand dieser Anmeldung und soll unabhängig von den anderen Merkmalen als offenbart und beanspruchbar mitgeteilt sein.

Eine andere Ausgestaltung der Vorrichtung sieht vor, dass ein Applikationskopf Mittel aufweist zum Abbilden von Strahlung in ein vorgegebenes Muster, insbesondere einen schmalen Strich, und zum Scannen (Bewegen) des Musters über die Haut. Dabei bleibt der Applikationskopf stationär und es bewegt sich nur die in dem Muster auf die Haut abgebildete Strahlung über die Haut. Auch diese Ausgestaltung der Erfindung soll hier als eigenständig unabhängig von den anderen Merkmalen der Erfindung offenbart und beanspruchbar angesehen werden.

Eine andere bevorzugte Ausgestaltung der Erfindung sieht vor, dass das Basisgerät und die zugeordneten unterschiedlichen Applikationsköpfe jeweils Mittel aufweisen, mit denen das Basisgerät einen angeschlossenen Applikationskopf "erkennen" kann und daraufhin entsprechend automatisch die Parameter (Stromversorgung, gegebenenfalls Strahlungssteuerung etc.) einstellt, sodass der Benutzer an dem Basisgerät keine besondere Einstellungen in Abhängigkeit von der Art des Applikationskopfes vornehmen muss. Zum Beispiel kann in jedem Applikationskopf ein Sender vorgesehen sein, der dann, wenn der Applikationskopf mit Strom versorgt wird, über die genannte flexible Leitung ein codiertes Signal an das Basisgerät abgibt, welches das Basisgerät lesen kann, um die Art des Applikationskopfes festzustellen, und daraufhin die erforderlichen und zugeordneten Steuerparameter für diesen Applikationskopf auswählt und den Benutzer auf einem Anzeigefeld anzeigt, was an Steuerung vorgesehen ist und was er gegebenenfalls noch einzustellen hat.

Nachfolgend werden Ausführungsbeispiele der Erfindung anhand der Zeichnung näher erläutert.

Es zeigt:
- Figur 1: ein erstes Ausführungsbeispiel einer Vorrichtung für dermatologische Behandlungen mit einem Basisgerät und einem Applikationskopf;
- Figur 2: ein zweites Ausführungsbeispiel einer Vorrichtung für dermatologische Behandlungen mit einem einzigen Basisgerät, an das zwei verschiedene Applikationsköpfe angeschlossen sind;
- Figur 3: ein weiteres Ausführungsbeispiel einer Vorrichtung für dermatologische Behandlungen mit einer Basisstation und einem Applikationskopf, der eine sogenannte Gesichtsmaske aufweist;
- Figur 4: eine Abwandlung der Vorrichtung gemäß Figur 2, wobei die Lichtquellen in den Applikationsköpfen angeordnet sind;
- Figur 5: eine abgewandelte Ausführungsform einer Vorrichtung für dermatologische Behandlungen mit einem Applikationskopf, in dem zwei unterschiedliche Lichtquellen angeordnet sind;
- Figur 6: ein weiteres Ausführungsbeispiel einer Vorrichtung für dermatologische Behandlungen mit einem Applikationskopf und darin angeordneten optischen Mitteln zum Abbilden von Strahlung in einen feinen Strich sowie Mitteln zum oszillierenden Bewegen des abgebildeten Strahlungsstriches über Haut;
- Figur 7: die Vorrichtung gemäß Figur 6 in anderer Darstellung ohne das Basisgerät;
- Figur 8: ein weiteres Ausführungsbeispiel einer Vorrichtung für dermatologische Behandlungen mit Einrichtungen zum Aufbringen von Creme oder Flüssigkeit auf die Haut;
- Figur 9: ein weiteres Ausführungsbeispiel einer Vorrichtung für dermatologische Behandlungen mit einer Vorrichtung zum Sprühen von flüssigen oder cremeartigen Mitteln auf Haut;
- Figur 10: eine Abwandlung der Vorrichtung gemäß Figur 8, wobei die Lichtquelle im Basisgerät angeordnet ist;
- Figur 11: eine weitere Variante einer Vorrichtung für dermatologische Behandlungen, wobei ein Applikationskopf eine Gesichtsmaske aufweist mit Sprühlöchern zum Auftragen von Flüssigkeit oder Creme auf die Haut; und
- Figur 12: eine Abwandlung der Ausführungsform gemäß Figur 9, wobei die Lichtquelle im Applikationskopf angeordnet ist.

Die Figuren zeigen schematisch die einzelnen Komponenten von Systemen für dermatologische Hautbehandlungen mit Strahlung. Einander entsprechende oder funktionsähnliche Bauteile sind mit gleichen Bezugszahlen versehen, gegebenenfalls ergänzt durch Buchstaben oder andere Unterscheidungsmerkmale.

Ein Basisgerät 10 ist über eine flexible Leitung 12 mit einem Applikationskopf 14 verbunden. In dem Applikationskopf 14 ist eine Lichtquelle 16, hier zum Beispiel eine LED 16a. Die von der Lichtquelle 16 emittierte Strahlung 20 ist auf Haut 18 gerichtet.

Das Basisgerät 10 beinhaltet die Stromversorgung auch für den Applikationskopf 14, elektronische Steuerungen für den Applikationskopf 14 und das Basisgerät selbst, und Bedienelemente für den Benutzer zum Einstellen von Parametern. Somit beinhaltet die flexible Leitung 12 insbesondere elektrische Leiter, gegebenenfalls (zum Beispiel wenn, wie bei weiter unten beschriebenen Ausführungsbeispielen, die Lichtquelle im Basisgerät angeordnet ist) Lichtleiter, und gegebenenfalls auch Kühlmittelleitungen etc.

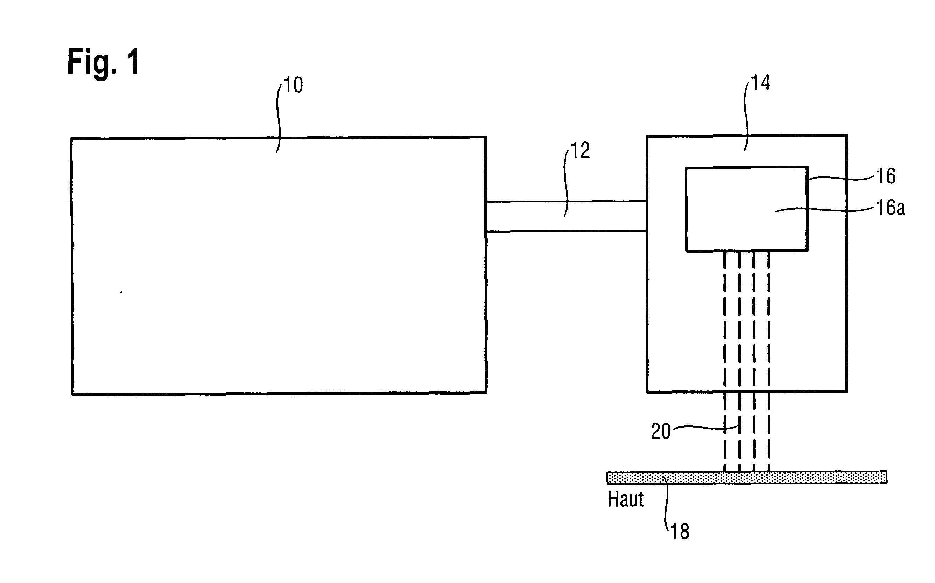

Beim Ausführungsbeispiel gemäß Figur 1 ist die Lichtquelle 16 im Applikationskopf 14 untergebracht. Dabei kann die Lichtquelle 16 insbesondere eine LED, ein Laser, oder eine hochenergetische Blitzlampe sein. Es können also entsprechend drei oder mehr unterschiedliche Applikationsköpfe 14 vorgesehen werden, die wahlweise an das Basisgerät 10 anschließbar sind und vom Basisgerät die erforderlichen Versorgungs- und Steuerungssignale erhalten. Jeder der verschiedenen Applikationsköpfe 14 wird also über eine dafür vorgesehene Verbindungsstelle am Basisgerät 10 über die jeweils zugeordnete Verbindungsleitung 12 an das Basisgerät angeschlossen.

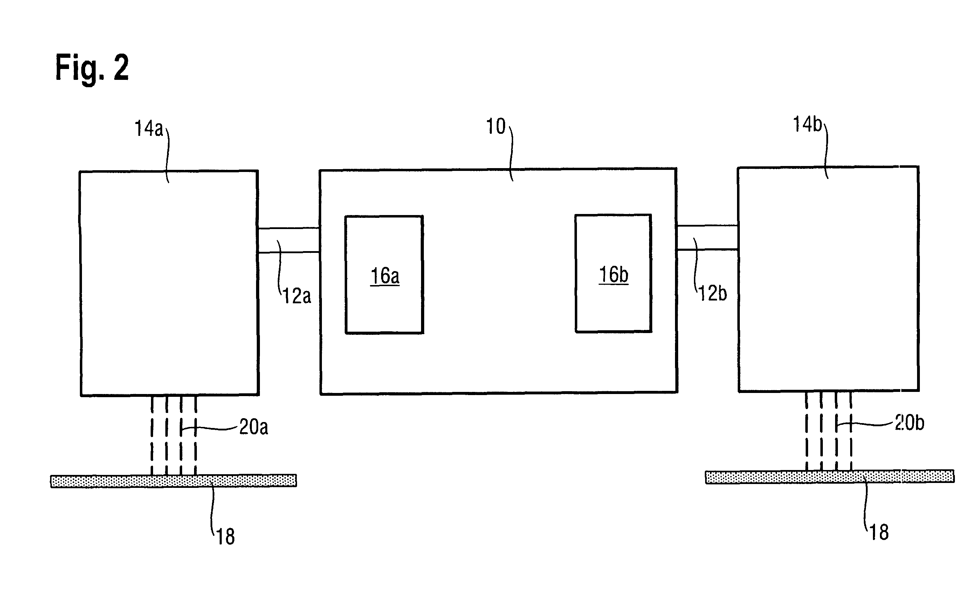

Figur 2 zeigt eine abgewandelte Ausführungsform mit einem Basisgerät 10, in dem als Lichtquelle eine LED 16a und als weitere Lichtquelle ein Laser 16b angeordnet sind. Über jeweils zugeordnete flexible Leitungen 12a bzw. 12b werden die emittierten Strahlungen der LED 16a bzw. des Lasers 16b zum Applikator übertragen. Damit ist es möglich, unterschiedliche Strahlungen 20a, 20b gleichzeitig oder nacheinander auf die Haut 18 aufzubringen.

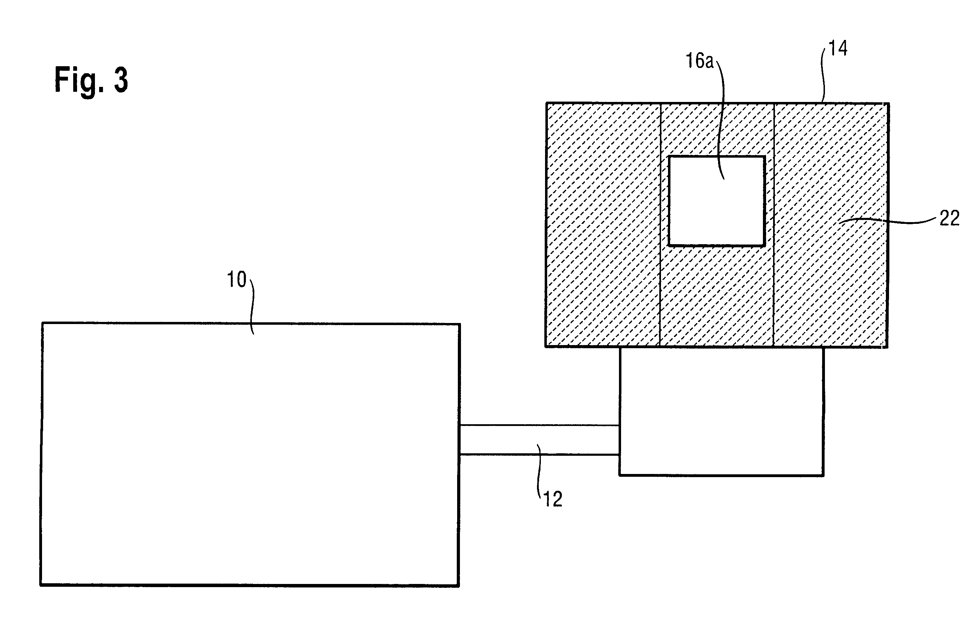

Figur 3 zeigt eine Abwandlung des Ausführungsbeispieles gemäß Figur 1, wobei eine sogenannte Gesichtsmaske ("face mask") am Applikationskopf 14 ausgeformt ist. Mit einer Gesichtsmaske können größere Körperteile genau bestrahlt werden. Dabei erfasst der Begriff "Gesichtsmaske" nicht nur das Gesicht im eigentlichen Sinn, sondern kann auch anderen Körperteilen, die behandelt werden sollen, entsprechen. Die Maske ist also der Form des zu behandelnden Körperteiles zumindest annähernd nachgebildet und ermöglicht so eine genau und reproduzierbare Positionierung des Applikationskopfes. In der Gesichtsmaske können mehrere Lichtquellen verteilt angeordnet sein, insbesondere LEDs oder Lampen. Ansonsten entspricht die Ausführungsform gemäß Figur 3 den Varianten gemäß den Figuren 1 und 2.

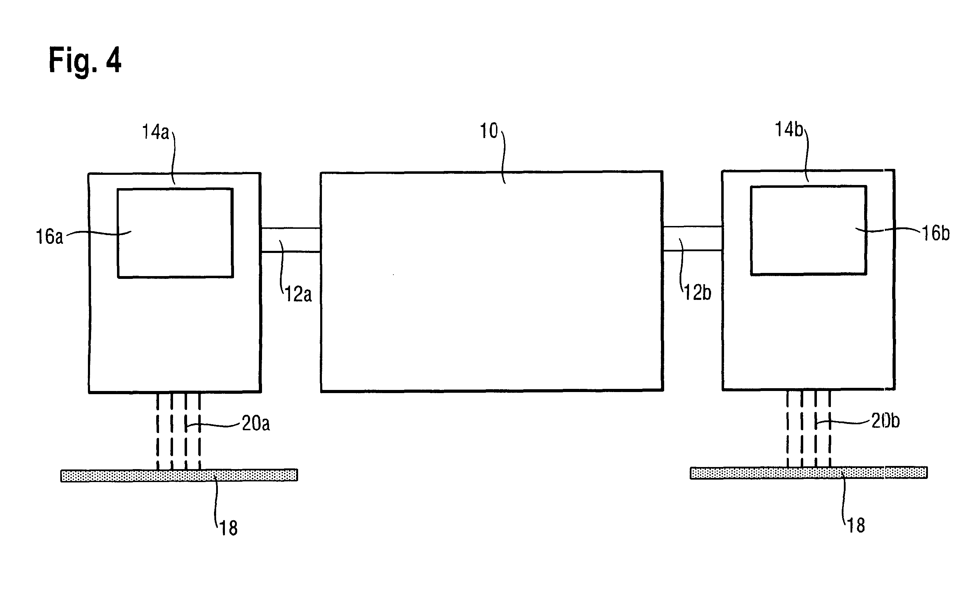

Figur 4 zeigt eine Abwandlung des Ausführungsbeispieles gemäß Figur 2 dahingehend, dass die Lichtquellen aus dem Basisgerät 10 in die Applikationsköpfe 14a, 14b verlegt sind. Im Applikationskopf 14a sind eine oder mehrere LEDs 16a angeordnet und im Applikationskopf 14b sind einer oder mehrere Laser 16b angeordnet. Ansonsten entsprechen die Ausgestaltungen den vorstehend beschriebenen Ausführungsbeispielen.

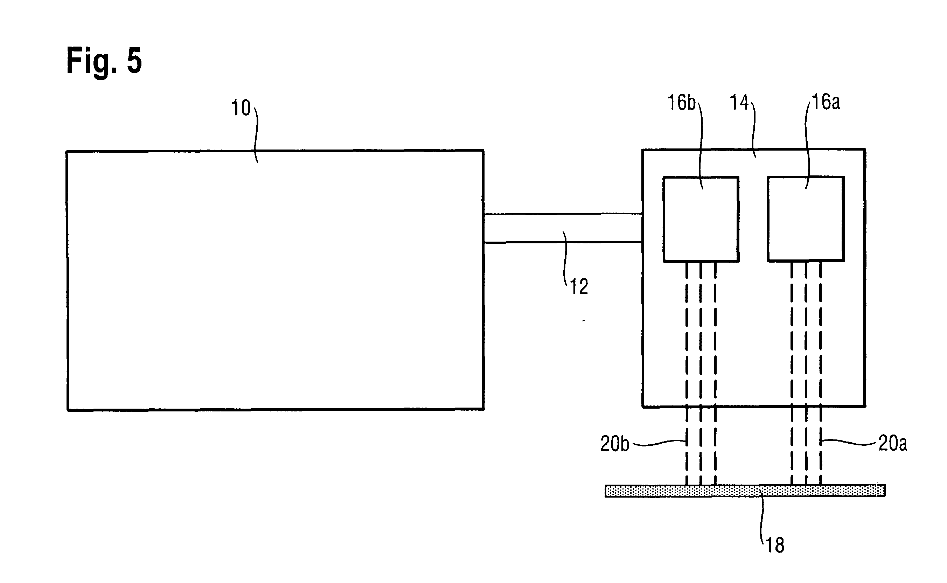

Figur 5 zeigt eine Variante einer Vorrichtung für dermatologische Behandlungen, bei der in einem Applikationskopf 14 zwei verschiedene Lichtquellen angeordnet sind, nämlich zum einen eine LED 16a (oder mehrere LEDs) und zum anderen ein (oder mehrere) Laser 16b. Die beiden Lichtquellen können wahlweise einzeln oder gemeinsam aktiviert werden.

Bei den anhand der Figuren 1 bis 5 beschriebenen Ausführungsbeispielen können insbesondere folgende Lichtquellen eingesetzt werden:

Als Laser ein IR-Diodenlaser, der bei 808 bzw. 980 nm emittiert. Solches Strahlung ist gut geeignet für die Epilation, je nach der gewählten Wellenlänge für helle Haut bzw. dunkle Haut.

Für die LEDs können zum Beispiel UV-LEDs gewählt werden, die bei 308 bzw. 370 nm emittieren. Diese Strahlung ist gut geeignet für die Behandlung von Autoimmunschwächen.

Einsetzbar sind auch blau emittierende LEDs zu Behandlung von Akne oder auch für die PDT. Gelb emittieren LEDs (zum Beispiel 590 nm) sind besonders geeignet für die Behandlung von Akne und die Hautverjüngung. Rot emittierende LEDs (640 nm) sind besonders geeignet für die Wundheilung und auch für die PDT.

Die vorstehend (und nachfolgend) beschriebenen Applikationsköpfe können auch mit Mitteln versehen sein, um Hautparameter zu ermitteln, zum Beispiel die Temperatur, die Hautbräunung, den Melaningehalt, die Dicke der Epidermis etc. Auch können Mittel vorgesehen sein zum erzeugen von Bildern der Behandlungsoberfläche.

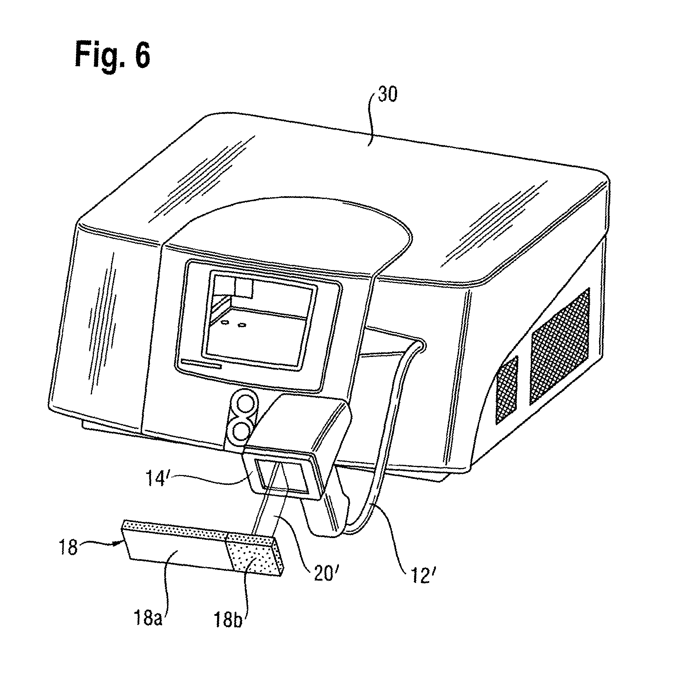

Figur 6 zeigt eine besondere Ausgestaltung eines Applikationskopfes 14', der mit optischen und elektrooptischen Mitteln versehen ist, um die von ihm abgegebene elektromagnetische Strahlung 20' auf der Haut 18 in einem schmalen Strich abzubilden. Der Strich kann zum Beispiel Querabmessungen von 0,5 bis5 mm haben, insbesondere 0,5 bis 2 mm. Seine Längsabmessung beträgt ein Vielfaches der Querabmessung, zum Beispiel das 2- bis 20-fache der Querabmessung.

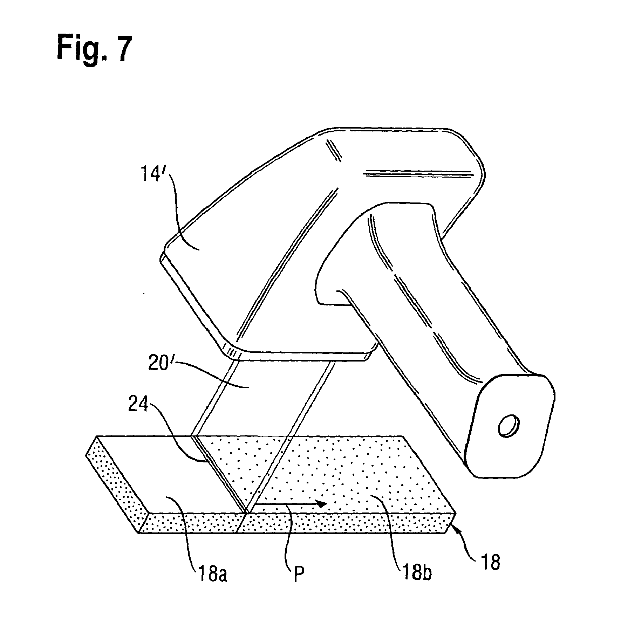

Figur 7 zeigt den Applikationskopf 14' von Figur 1 aus anderer Perspektive in Richtung auf die Oberfläche der zu bestrahlenden Haut 18 gesehen. Wie dargestellt ist, erzeugen die optischen Mittel im Applikationskopf 14' eine Strahlung 20', die auf der Haut 18 ein strichförmiges Muster 24 abbildet. Der Strich 24 wird in Richtung des Pfeiles P gescannt. Der Strich 24 behält also während des Scannens seine Form und Abmessung und wird in Richtung des Pfeiles über die Haut 18 bewegt. Der bereits behandelte Abschnitt der Haut ist in Figur 7 mit 18a bezeichnet und der noch zu behandelnde Abschnitt der Haut mit 18b.

Diese Anordnung ermöglicht es, mit relativ kleinen Spotgrößen zu operieren, ohne eine für eine Follikelerhitzung erforderliche Tiefenwirkung zu verringern. Durch die kontinuierliche Bestrahlung erfolgt eine hinreichende Erhitzung und ein Kummulieren der Wärme in der Haut. Es können relativ schwächere CW-Laser eingesetzt und optimal ausgenutzt werden. Aufgrund der Fokussierung in einem Strich sinkt auch die erforderliche CW-Leistung. Es können Diodenlaser und Festkörperlaser eingesetzt werden. Dabei sind die anhand der Figuren 1 bis 5 beschriebenen Varianten der Anordnung der Lichtquellen im System möglich.

Aufgrund des kontinuierlichen Abfahrens des zu epillierenden Hautbereiches mit dem Scanner können zum Beispiel relativ schwache Diodenlaser (zum Beispiel mit 300 W) zur schnellen Epilation eingesetzt werden. Durch des fehlende An- und Ausschalten der Strahlung kann ein größerer Prozentteil (mehr als 50 %) der überhaupt zur Verfügung stehenden CW-Laserleistung für die Epilation eingesetzt werden, was insgesamt zu einer höheren Behandlungsgeschwindigkeit führt. Die genannten kleinen Spotabmessungen (Länge mal Breite des Striches) ermöglichen kleine, preisgünstige und einfach zu kühlende sowie mit relativ schwacher Leistung zu versorgenden Laser-Diodenmodule, ohne die Bedienerfreundlichkeit und auch die Eindringtiefe in die Haut nachteilig zu beeinflussen. Es sind kleine, handliche und gut transportable Geräte dargestellbar.

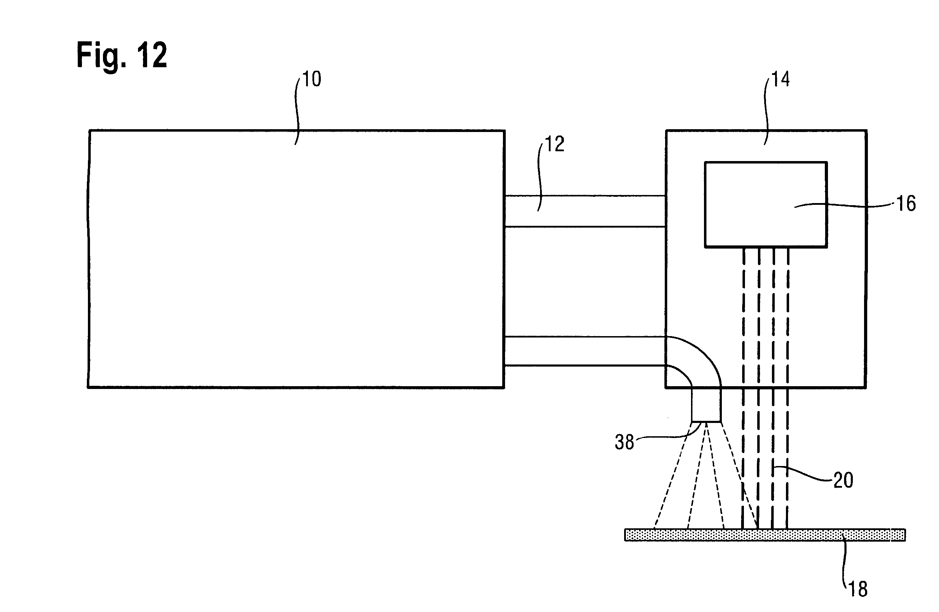

Im Unterschied zum Stand der Technik gemäß US 6,273,884, dort Figur 12A, erfolgt beim anhand der Figuren 6 und 7 erläuterten Ausführungsbeispiel der Vorrichtung das Scannen senkrecht zur Längsrichtung des Striches (Richtung des Pfeiles P).

Beim Ausführungsbeispiel gemäß den Figuren 6 und 7 wird bevorzugt Laserlicht mit Wellenlängen im Bereich zwischen 650 und 1200 nm eingesetzt. Hat der Strich 24 zum Beispiel Abmessungen von 0,3 cm x 1 cm, dann können die geometrischen Parameter des Scannens so eingestellt werden, dass zum Beispiel eine Hautbehandlungsfläche von 5 cm x 1 cm abgefahren (gescannt) wird, wobei die Abmessung von -1 cm derjenigen der Längsabmessung des Strichs 24 entspricht. Die Scannung ist so gesteuert, dass die Behandlungsfläche durch den im Verhältnis zu ihr wesentlich kleineren Strich 24 kontinuierlich überstrichen wird, d.h. die Lichteinstrahlung wird während des Scannens nicht an- und ausgeschaltet. Dies bedeutet praktisch, dass mit sehr langen Pulsdauern, zum Beispiel Pulsdauern im Bereich von mehr als 200 ms bis 2 s gearbeitet wird und während dieser Pulsdauern wird andauernd Licht auf die Haut eingestrahlt. Das kontinuierliche Abscannen wird so gesteuert, dass eine Gewebestelle nur für ca. 5 bis 50 ms mit Licht bestrahlt wird, um Verbrennungen zu vermeiden. Die übrigen relevanten Parameter, wie Pulsdauer, Fluenz und Scanbereich lassen sich vom Benutzer unter Berücksichtigung der genannten Randbedingungen einstellen.

Als Lichtquellen kommen Laser, insbesondere CW-strahlende Laser, wie zum Beispiel Diodenlaser, in Betracht, aber auch LEDs einschließlich OLEDs. Geeignet sind auch Lampen, deren Licht in der beschriebenen Weise fokussierbar ist.

Hinsichtlich der Anordnung der Lichtquellen sind die anhand der Figuren 1 bis 5 beschriebenen Varianten möglich. Scanner und deren Ansteuerungen sind als solches im Stand der Technik gut bekannt, sie arbeiten mit beweglichen Spiegeln und Linsen. Alternativ könnte auch eine im Applikationskopf 14' angeordnete leichte Lichtquelle selbst bewegt werden, um den Scanvorgang zu erzielen.

Mit den oben beschriebenen Leistungs- und Zeitparametern können Haarfollikel in tieferen Hautschichten (ca. 500 µm-4 mm) erreicht und erhitzt werden.

Statt eines strengen CW-Betriebes reicht auch ein quasi CW-Betrieb, d.h. es können kurze Pulsdauern größer als 1 ms mit vergleichsweisen kurzen Pulspausen eingesetzt werden, die kleiner sind als die Pulse selbst oder es können auch sehr kurze Pulse kleiner als 0,5 ms eingesetzt werden mit Pulspausen im gleichen Zeitbereich.

Die vorstehend beschriebenen Vorrichtungen weisen bevorzugt Mittel auf, mit denen die für die Kühlung und/oder Lichterzeugung verwendeten Energiemengen zeitlich so veränderbar sind, dass die Durchschnitts- und/oder Spitzenlichtleistungen bei vorgegebener Stromeingangsleistung (dies ist der sogenannte Anschlusswert) maximiert werden.

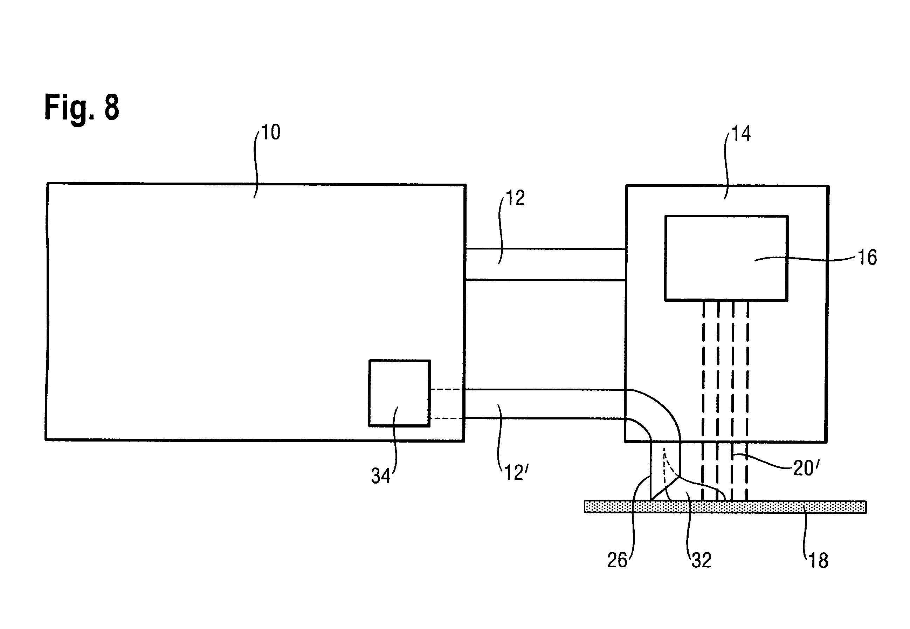

Figur 8 zeigt eine Ausgestaltung mit einem Basisgerät 10 und einem Applikator 14 sowie einer flexiblen Leitung 12' zum Übertragen einer Creme oder einer Flüssigkeit aus einem Reservoir 34 zum Applikator 14. Aus dem Applikator 14 steht ein Tropfenspender 26 vor, der einen Tropfen 32 auf die Haut 18 aufbringt. Der Applikationskopf ermöglicht also die Kombination einer Lichtbehandlung mit topisch aufzutragenden pharmazeutischen oder pflegenden Substanzen. Die Vorrichtung ermöglicht es, kurz vor, während oder kurz nach einer Lichtbehandlung Substanzen in flüssiger Form, insbesondere Cremes, Salben oder Spray, oder in gasförmiger Form auf die Haut aufzubringen, um die Behandlung zu fördern oder Nebenwirkungen zu verringern.

Die in den Figuren 8 bis 12 beschriebenen Varianten der Vorrichtung für dermatologische Behandlungen ermöglichen es, pharmazeutisch wirksame oder pflegende Substanzen im wesentlichen zeitlich und räumlich parallel zur Lichtbehandlung aufzubringen.

Für den Operateur ist diese kombinierte Behandlung mit den Systemen gemäß den Figuren 8 bis 12 in einfacher Weise möglich.

In Abwandlung der beschriebenen Ausführungsbeispiele kann über eine Leitung analog der flexiblen Leitung 12' auch Kühlluft auf die Haut aufgebracht werden.

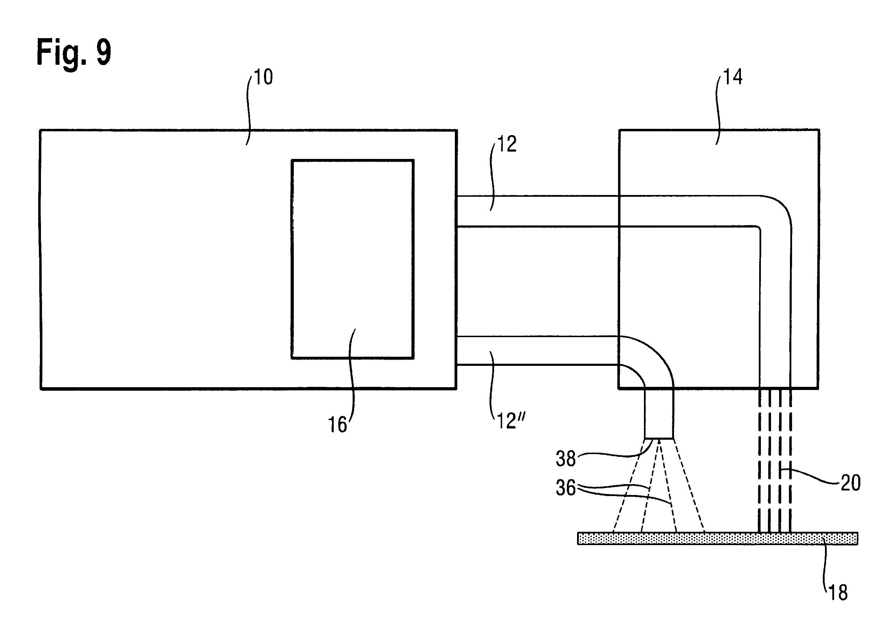

Figur 9 zeigt eine Variante, mit einer Sprühvorrichtung. Aus einer Düse 38 wird Sprühmittel 36 auf die Haut 18 aufgebracht. Bezüglich der Substanzen gilt das anhand der Figur 8 Gesagte.

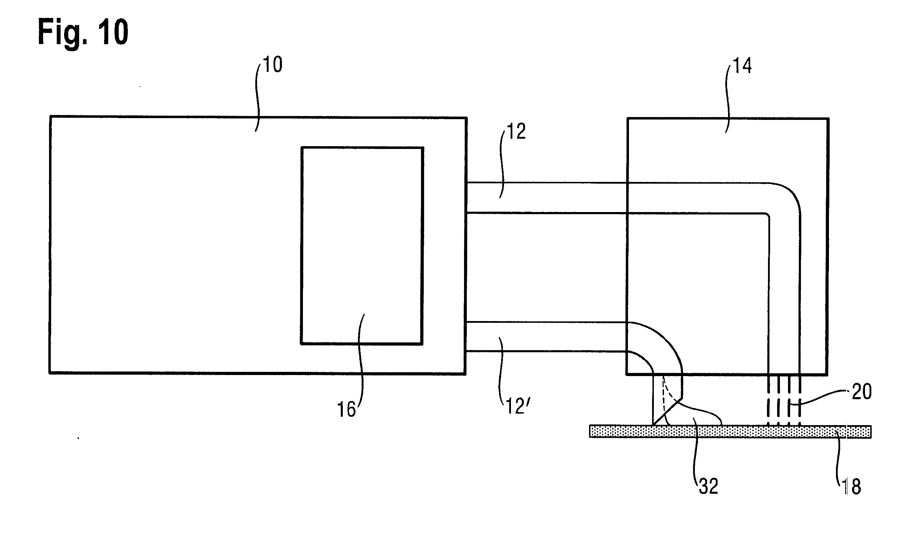

Figur 10 zeigt eine Abwandlung der Vorrichtung gemäß Figur 8 mit einer Lichtquelle 16 im Basisgerät 10.

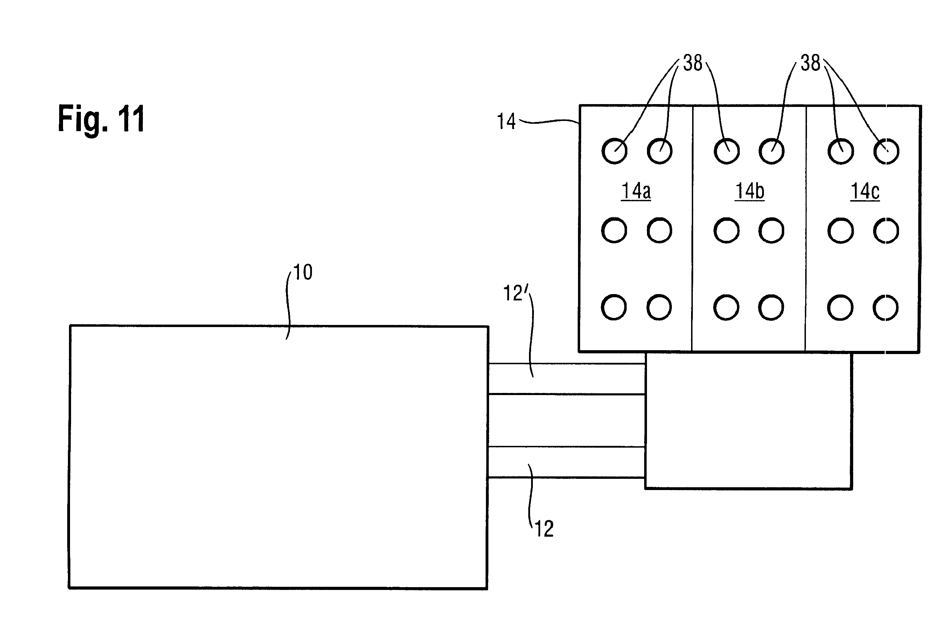

Figur 11 zeigt eine Ausgestaltung eines Applikationskopfes 14 mit einer sogenannten Gesichtsmaske (siehe oben). Pharmazeutische und/oder pflegende Substanzen können über eine Vielzahl von Düsen 38, die großflächig über die Gesichtsmaske verteilt sind, zeitlich und mengenmäßig definiert gesteuert auf die Haut aufgebracht werden.

Figur 12 zeigt eine Abwandlung des Ausführungsbeispieles gemäß Figur 8 mit einer Sprühvorrichtung (Düse 38) anstelle des eine Flüssigkeit oder einen Tropfen direkt auf die Haut auftragenden Spenders.

In den Ausführungsbeispielen gemäß den Figuren 8 bis 12 kann das Reservoir für die pflegende oder pharmazeutisch wirksame Substanz in Abwandlung der vorstehenden Beschreibung auch im Applikationskopf oder auch in einer Gesichtsmaske selbst angeordnet sein. In diesen Fällen kann auf die Leitung 12' verzichtet werden.

## Patentansprüche

1. Vorrichtung für dermatologische Behandlungen mit einem Basisgerät (10), an das wahlweise über flexible Leitungen (12) zumindest ein erster Applikationskopf (14a) zum Aufbringen von Strahlung (20) einer LED (16a) auf Haut, und/oder zumindest
ein zweiter Applikationskopf (14b) zum Aufbringen von Strahlung eines Lasers (16b) oder einer hochenergetischen Blitzlampe (16c) auf Haut (18) anschließbar sind.

2. Vorrichtung nach Anspruch 1, **dadurch gekennzeichnet, dass** die LED (16a) und/oder der Laser (16b) in einem Applikationskopf (14) angeordnet sind.

3. Vorrichtung nach Anspruch 1, **dadurch gekennzeichnet, dass** die LED (16a) und/oder der Laser (16b) und/oder die Blitzlampe (16c) in dem Basisgerät (10) angeordnet sind.

4. Vorrichtung nach einem der vorhergehenden Ansprüche, **dadurch gekennzeichnet, dass** die LED (16a) schmalbandig emittiert , insbesondere mit einer Spektralbreite kleiner als 50 nm.

5. Vorrichtung nach einem der vorhergehenden Ansprüche, **dadurch gekennzeichnet, dass** die LED (16a) mit Intensitäten emittiert, die ausgelegt sind zur Erzielung von fotobiologischen oder fotochemischen Effekten in der Haut.

6. Vorrichtung nach einem der vorhergehenden Ansprüche, **dadurch gekennzeichnet, dass** die LED (16a) im UVA-Bereich emittiert.

7. Vorrichtung nach einem der Ansprüche 1 bis 5, **dadurch gekennzeichnet, dass** die LED (16a) im UVB-Bereich emittiert.

8. Vorrichtung nach einem der vorhergehenden Ansprüche mit einem LED-Applikationskopf (14), der zwei oder mehr LEDs unterschiedlicher Wellenlängen aufweist, insbesondere zur homogenen Beleuchtung einer Hautfläche.

9. Vorrichtung nach Anspruch 7, **dadurch gekennzeichnet, dass** unterschiedliche LEDs in einem Applikationskopf (14) wahlweise einsetzbar sind.

10. Vorrichtung nach Anspruch 1, **dadurch gekennzeichnet, dass** der Laser ein Festkörperlaser, insbesondere ein IR-Laser, mit Intensitäten zur Erzielung von fotothermischen Effekten in der Haut ist.

11. Vorrichtung nach Anspruch 10, **dadurch gekennzeichnet, dass** der Festkörperlaser ein CW-Laser, insbesondere ein Diodenlaser, ist.

12. Vorrichtung nach einem der vorhergehenden Ansprüche mit einem Applikationskopf (14), der eine Einrichtung (38) aufweist zum Aufbringen einer Flüssigkeit oder von Creme auf Haut, insbesondere eine Einrichtung zum Aufsprühen und/oder Auftropfen einer Flüssigkeit oder eines Cremes.

13. Vorrichtung nach einem der vorhergehenden Ansprüche mit einem Applikationskopf (14), der optische Mittel aufweist zum Abbilden von Strahlung (20) in ein vorgegebenes Muster, und einen Scanner zum Bewegen des abgebildeten Strahls über Haut.

14. Vorrichtung nach Anspruch 13, **dadurch gekennzeichnet, dass** das Muster linienförmig ist.

15. Vorrichtung nach Anspruch 14, **gekennzeichnet durch** Mittel zum Bewegen des linienförmigen Musters senkrecht zu dessen Längsachse.

16. Vorrichtung nach einem der Ansprüche 13 bis 15, **dadurch gekennzeichnet, dass** das Muster rechteckförmig ist mit einem Verhältnis von langer zu kurzer Seite größer 2.

17. Vorrichtung nach einem der vorhergehenden Ansprüche, **dadurch gekennzeichnet, dass** das Basisgerät (10) und/oder die zugeordneten Applikationsköpfe (14) Mittel aufweisen, mit denen das Basisgerät einen angeschlossenen Applikationskopf erkennen kann.

## Geänderte Patentansprüche

### Geänderte Patentansprüche gemäss Regel 137(2) EPÜ.

**1.** Vorrichtung für dermatologische Behandlungen mit einem Basisgerät (10), an das wahlweise über flexible Leitungen (12) zumindest ein erster Applikationskopf (14a) zum Aufbringen von Strahlung (20) einer LED (16a) auf Haut, und/oder zumindest
ein zweiter Applikationskopf (14b) zum Aufbringen von Strahlung eines Lasers (16b) oder einer hochenergetischen Blitzlampe (16c) auf Haut (18) anschließbar sind,
**dadurch gekennzeichnet, dass** zumindest ein Applikationskopf (14) optische Mittel zum Abbilden von Strahlung (20) in ein vorgegebenes Muster und einen Scanner zum Bewegen des abgebildeten Strahls über die Haut aufweist.

**2.** Vorrichtung nach Anspruch 1, **dadurch gekennzeichnet, dass** das Muster linienförmig ist.

**3.** Vorrichtung nach Anspruch 1 oder 2, **gekennzeichnet durch** Mittel zum Bewegen des linienförmigen Musters senkrecht zu dessen Längsachse.

**4.** Vorrichtung nach einem der Ansprüche 1 bis 3, **dadurch gekennzeichnet, dass** das Muster rechteckförmig ist mit einem Verhältnis von langer zu kurzer Seite größer 2.

**5.** Vorrichtung nach einem der vorhergehenden Ansprüche, **dadurch gekennzeichnet, dass** die LED (16a) und/oder der Laser (16b) in einem Applikationskopf (14) angeordnet sind.

**6.** Vorrichtung nach einem der vorhergehenden Ansprüche, **dadurch gekennzeichnet, dass** die LED (16a) und/oder der Laser (16b) und/oder die Blitzlampe (16c) in dem Basisgerät (10) angeordnet sind.

**7.** Vorrichtung nach einem der vorhergehenden Ansprüche, **dadurch gekennzeichnet, dass** die LED (16a) schmalbandig emittiert, insbesondere mit einer Spektralbreite kleiner als 50 nm.

**8.** Vorrichtung nach einem der vorhergehenden Ansprüche, **dadurch gekennzeichnet, dass** die LED (16a) mit Intensitäten emittiert, die ausgelegt sind zur Erzielung von fotobiologischen oder fotochemischen Effekten in der Haut.

**9.** Vorrichtung nach einem der vorhergehenden Ansprüche, **dadurch gekennzeichnet, dass** die LED (16a) im UVA-Bereich emittiert.

**10.** Vorrichtung nach einem der Ansprüche 1 bis 8, **dadurch gekennzeichnet, dass** die LED (16a) im UVB-Bereich emittiert.

**11.** Vorrichtung nach einem der vorhergehenden Ansprüche mit einem LED-Applikationskopf (14), der zwei oder mehr LEDs unterschiedlicher Wellenlängen aufweist, insbesondere zur homogenen Beleuchtung einer Hautfläche.

**12.** Vorrichtung nach einem der vorhergehenden Ansprüche, **dadurch gekennzeichnet, dass** unterschiedliche LEDs in einem Applikationskopf (14) wahlweise einsetzbar sind.

**13.** Vorrichtung nach Anspruch 1, **dadurch gekennzeichnet, dass** der Laser ein Festkörperlaser, insbesondere ein IR-Laser, mit Intensitäten zur Erzielung von fotothermischen Effekten in der Haut ist.

**14.** Vorrichtung nach Anspruch 13, **dadurch gekennzeichnet, dass** der Festkörperlaser ein CW-Laser, insbesondere ein Diodenlaser, ist.

**15.** Vorrichtung nach einem der vorhergehenden Ansprüche mit einem Applikationskopf (14), der eine Einrichtung (38) aufweist zum Aufbringen einer Flüssigkeit oder von Creme auf Haut, insbesondere eine Einrichtung zum Aufsprühen und/oder Auftropfen einer Flüssigkeit oder eines Cremes.

**16.** Vorrichtung nach einem der vorhergehenden Ansprüche, **dadurch gekennzeichnet, dass** das Basisgerät (10) und/oder die zugeordneten Applikationsköpfe (14) Mittel aufweisen, mit denen das Basisgerät einen angeschlossenen Applikationskopf erkennen kann.
